(19) Europäisches Patentamt
European Patent Office
Office européen des brevets

(11) EP 1 715 743 B1

(12) EUROPÄISCHE PATENTSCHRIFT

(45) Veröffentlichungstag und Bekanntmachung des Hinweises auf die Patenterteilung:
**18.09.2013 Patentblatt 2013/38**

(21) Anmeldenummer: **05701229.6**

(22) Anmeldetag: **28.01.2005**

(51) Int Cl.:
***A01N 47/40*** (2006.01) ***A01N 55/10*** (2006.01)
***A01N 53/10*** (2006.01) ***A01N 53/08*** (2006.01)
***A01N 53/06*** (2006.01) ***A01N 47/02*** (2006.01)
***A01N 37/44*** (2006.01) ***A01N 37/38*** (2006.01)
***A01N 31/14*** (2006.01) *A01N 47/40* (2006.01)
*A01N 55/10* (2006.01) *A01N 53/10* (2006.01)
*A01N 53/08* (2006.01) *A01N 53/06* (2006.01)
*A01N 47/02* (2006.01) *A01N 37/44* (2006.01)
*A01N 37/38* (2006.01) *A01N 31/14* (2006.01)

(86) Internationale Anmeldenummer:
**PCT/EP2005/000830**

(87) Internationale Veröffentlichungsnummer:
**WO 2005/077186 (25.08.2005 Gazette 2005/34)**

(54) **INSEKTIZIDE WIRKSTOFFKOMBINATIONEN AUS THIACLOPRID UND PYRETHROIDEN**

COMBINATIONS OF INSECTICIDE ACTIVE AGENTS BASED ON THIACLOPRID AND PYRETHROIDS

COMBINAISONS D'AGENTS ACTIFS INSECTICIDES A BASE DE THIACLOPRIDE ET DE PYRETHROIDES

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**HR**

(30) Priorität: **10.02.2004 DE 102004006324**

(43) Veröffentlichungstag der Anmeldung:
**02.11.2006 Patentblatt 2006/44**

(73) Patentinhaber: **Bayer CropScience Aktiengesellschaft**
**40789 Monheim (DE)**

(72) Erfinder:
- **EBBINGHAUS, Dirk**
  **42111 Wuppertal (DE)**
- **HINTERHUBER, Andreas**
  **51467 Bergisch Gladbach (DE)**
- **THIELERT, Wolfgang**
  **51519 Odenthal (DE)**
- **HUNGENBERG, Heike**
  **40764 Langenfeld (DE)**
- **WECKWERT, Holger**
  **42799 Leichlingen (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**WO-A-02/30200**
**WO-A-02/43494**
**WO-A-99/65313**
**WO-A-02/087338**
**WO-A-03/070000**
**FR-A- 2 784 011**
**US-A- 5 661 164**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 715 743 B1

## Beschreibung

Die vorliegende Erfindung betrifft neue Wirkstoffkombinationen, die aus Thiacloprid einerseits und weiteren bekannten insektiziden Wirkstoffen andererseits bestehen und sehr gut zur Bekämpfung von tierischen Schädlingen geeignet sind.

Es ist bereits bekannt, dass Thiacloprid der Formel

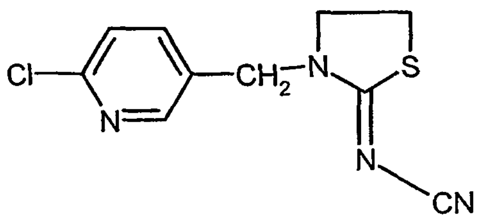

insektizide Eigenschaften besitzt (EP 0 235 725).

Weiterhin ist schon bekannt, dass Pyrethroide insektizide Eigenschaften besitzen (vgl. WO 93-22 297, WO 93-10 083, DE-A 2 641 343, EP-A-347 488, EP-A-210 487, US-A 3 264 177 und EP-A-234 045). Allerdings ist die Wirkung dieser Stoffe nicht immer befriedigend.

Es ist ebenfalls bekannt, dass Wirkstoffkombinationen aus Chloronikotinyl-Insektiziden mit Pyrethroiden eine synergistische Wirkung gegen Schädlinge aufweisen (vgl. WO 02-43494, WO 02-087338, WO 02-30200, FR-A-2 784 011 und US-A-5 661 164).

Es wurde nun gefunden, dass Mischungen enthaltend Thiacloprid und

2. Alpha-cypermethrin

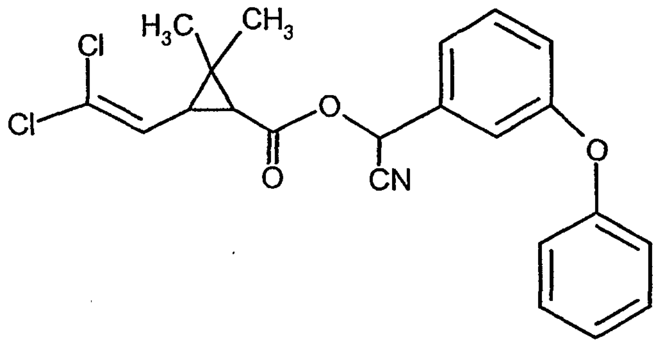

bekannt ans EP-A-067 461 und/oder

6. Deltamethrin

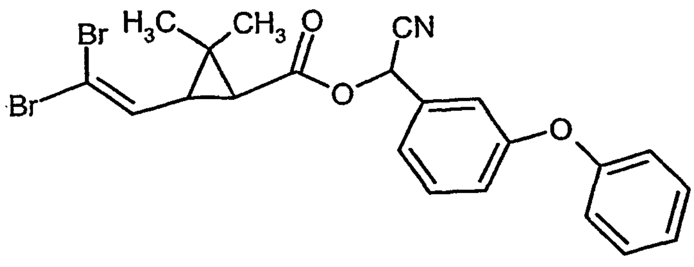

bekannt aus DE-A-2 326 077

und/oder

12. Lambda-cyhalothrin

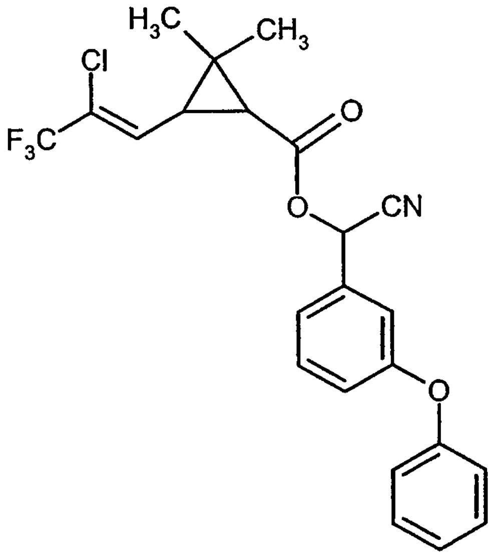

bekannt aus EP-A-106 469
und/oder
14. Taufluvalinat

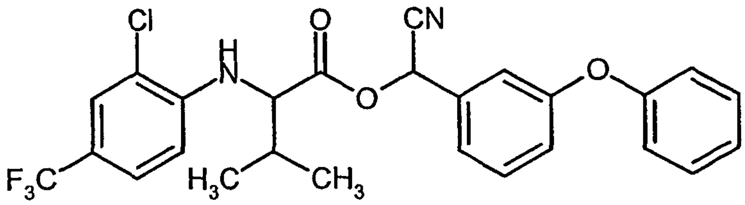

bekannt aus EP-A-038 617
und/oder
16. Zeta-cypermethrin

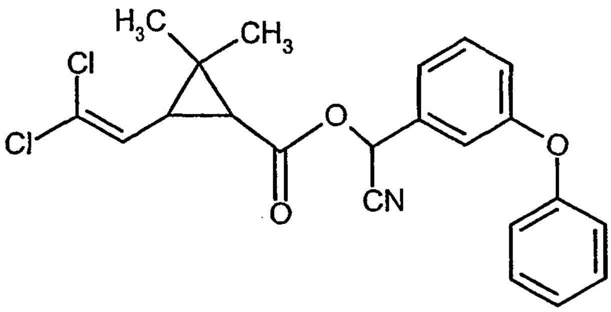

bekannt aus EP-A-026542
sehr gute insektizide Eigenschaften besitzen.

Überraschenderweise ist die, insbesondere insektizide, Wirkung der erfindungsgemäßen Wirkstoffkombination wesentlich höher als die Summe der Wirkungen der einzelnen Wirkstoffe. Es liegt ein nicht vorhersehbarer echter synergistischer Effekt vor und nicht nur eine Wirkungsergänzung.

Die erfindungsgemäßen Wirkstoffkombinationen enthalten neben Thiacloprid mindestens einen Wirkstoff der Verbindungen 2, 6, 12, 14 und 16.

Die Wirkstoffkombinationen können darüber hinaus auch weitere fungizid, akarizid oder insektizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich der synergistische Effekt besonders deutlich. Jedoch können die Gewichtsverhältnisse der Wirkstoffe in den Wirkstoffkombinationen in einem relativ großen Bereich variiert werden. Im Allgemeinen enthalten die

erfindungsgemäßen Kombinationen Thiacloprid und den Mischpartner in den in der nachfolgenden Tabelle angegeben bevorzugten und besonders bevorzugten Mischungsverhältnissen:

* die Mischungsverhältnisse basieren auf Gewichtsverhältnissen. Das Verhältnis ist zu verstehen als Thiacloprid: Mischpartner

| Mischpartner | bevorzugtes Mischungs-verhältnis | besonders bevorzugtes Mischungsverhältnis |
|---|---|---|
| Alpha-Cypermethrin | 125:1 bis 1:25 | 1:1 bis 1:25 |
| Deltamethrin | 125:1 bis 1:25 | 1:1 bis 1:25 |
| Lambda-Cyhalothrin | 125:1 bis 1:25 | 1:1 bis 1:25 |
| Tau-fluvalinat | 125:1 bis 1:25 | 1:1 bis 1:25 |
| Zeta-Cypermethrin | 125:1 bis 1:25 | 1:1 bis 1:25 |

Die erfindungsgemäßen Wirkstoffkombinationen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren und Nematoden, die in der Landwirtschaft, in Forsten, in Gärten und Freizeiteinrichtungen, im Vorrats-und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp.

Aus der Ordnung der Phthiraptera z.B. Pediculus humanus corporis, Haematopinus spp., Linognathus spp., Trichodectes spp., Damalinia spp.

Aus der Ordnung der Thysanoptera z.B. Hercinothrips femoralis, Thrips tabaci, Thrips palmi, Frankliniella accidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes

spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.

Aus der Ordnung der Siphonaptera z.B. Xenopsylla cheopis, Ceratophyllus spp.

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Als Kulturpflanzen seien Getreide (Weizen, Hafer, Gerste, Roggen, Reis), Mais, Soja, Kartoffel, Baumwolle, Tabak, Raps sowie Obstpflanzen (mit den Früchten Äpfel, Birnen, Zitrusfrüchten und Weintrauben) erwähnt. Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstrchen, Injizieren und bei Vermehrungsmaterial, insbesondere bei Samen, weiterhin durch ein- oder mehrschichtiges Umhüllen.

Die Wirkstoffkombinationen können in die üblichen Formulierungen überführt werden, wie Lösungen, Emulsionen, Spritzpulver, Suspensionen, Pulver, Stäubemittel, Pasten, lösliche Pulver, Granulate, Suspensions- Emulsions-Konzentrate, Wirkstoff- imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/ oder schaumerzeugende Mittel kommen in Frage: z.B. nichtionogene und anionische Emulgatoren, wie Polyoxyethylen- Fettsäure- Ester, Polyoxyethylen- Fettalkohol- Ether, z.B. Alkylaryl- polyglykolether, Alkylsulfonate, Alkylsulfate, Arylsulfonate sowie Einweißhydrolysate; als Dispergiermittel kommen in Frage: z.B. Lignin- Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spuren-nährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffkombinationen können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasser-stoffe, Phenylharnstoffe, durch Mikroorganismen hergestellte Stoffe u.a.

Besonders günstige Mischpartner sind z.B. die folgenden:

Fungizide:

2- Phenylphenol; 8- Hydroxyquinoline sulfate; Acibenzolar- S- methyl; Aldimorph; Amidoflumet; Ampropylfos; Ampropylfos- potassium; Andoprim; Anilazine; Azaconazole; Azoxystrobin; Benalaxyl; Benodanil; Benomyl; Benthiavalicarb- isopropyl; Benzamacril; Benzamacril- isobutyl; Bilanafos; Binapacryl; Biphenyl; Bitertanol; Blasticidin- S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole- M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl- Al; Fosetylsodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris (albesilate) ; Iodocarb; Ipconazole; Iprobenfos; Iprodione; Iprovalicarb; Irumamycin; Isoprothiolane; Isovaledione; Kasugamycin; Kresoxim- methyl; Mancozeb; Maneb; Meferimzone; Mepanipyrim; Mepronil; Metalaxyl; Metalaxyl- M; Metconazole; Methasulfocarb; Methfuroxam; Metiram; Metominostrobin; Metsulfovax; Mildiomycin; Myclobutanil; Myclozolin; Natamycin; Nicobifen; Nitrothalisopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine- sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate- methyl; Thiram; Tioxymid; Tolclofos- methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)- N- [2- [4- [[3- (4- chlorophenyl)- 2- propynyl] oxy]- 3- methoxyphenyl]- ethyl]- 3- methyl- 2- [(methylsulfonyl) amino]- butanamide; 1- (1- naphthalenyl)- 1H- pyrrole- 2, 5- dione; 2, 3, 5, 6- tetrachloro- 4- (methylsulfonyl)- pyridine; 2- amino- 4- methyl- N- phenyl- 5- thiazolecarbox- amide; 2- chloro- N- (2, 3- dihydro- 1, 1, 3- trimethyl- 1H- inden- 4- yl)- 3- pyridincarboxamide; 3, 4, 5- tri- chloro- 2, 6- pyridinedicarbonitrile; Actinovate; cis- 1- (4- chlorophenyl)- 2- (1H- 1, 2, 4- triazole- 1- yl)- cycloheptanol; methyl 1- (2, 3- dihydro- 2, 2- dimethyl- 1H- inden- 1- yl)- 1H- imidazole- 5- carboxylate; monopotassium carbonate; N- (6- methoxy- 3- pyridinyl)- cyclopropanecarboxamide; N- butyl- 8- (1, 1- dimethylethyl)- 1- oxaspiro [4.5] decan- 3- amine; Sodium tetrathiocarbonate;
sowie Kupfersalze und- zubereitungen, wie Bordeaux mixture; Copper hydroxide; Copper naphthenate; Copper oxychloride; Copper sulfate; Cufraneb; Cuprous oxide; Mancopper; Oxinecopper.

Bakterizide:

Bronopol, Dichlorophen, Nitrapyrin, Nickel- Dimethyldithiocarbamat, Kasugamycin, Octhilinon, Furancarbonsäure, Oxytetracyclin, Probenazol, Streptomycin, Tecloftalam, Kupfersulfat und andere Kupfer- Zubereitungen.

Insektizide / Akarizide / Nematizide

1. Acetylcholinesterase (AChE) Inhibitoren

1.1 Carbamate, zum Beispiel
Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb
Triazamate

1.2 Organophosphate, zum Beispiel
Acephate, Azamethiphos, Azinphos (- methyl, -ethyl) , Bromophos- ethyl, Brom- fenvinfos (- methyl) , Butathiofos, Cadusafos, Carbophenothion, Chlorethoxyfos, Chlorfenvinphos, Chlormephos, Chlorpyrifos (- methyl/- ethyl) , Coumaphos, Cyanofenphos, Cyanophos, Chlorfenvinphos, Demeton- S- methyl, Demeton- S- methylsulphon, Dialifos, Diazinon, Dichlofenthion, Dichlorvos/ DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfo- thion, Fenthion, Flupyrazofos, Fonofos, Formothion, Fosmethilan, Fosthiazate, Heptenophos, Iodofenphos, Iprobenfos, Isazofos, Isofenphos, Isopropyl O- salicylate, Isoxathion, Malathion, Mecarbam, Methacrifos, Methamidophos, Methidathion, Mevinphos, Monocrotophos, Naled, Omethoate, Oxydemeton- methyl, Parathion (- methyl/- ethyl) , Phenthoate, Phorate, Phosalone, Phosmet, Phosphamidon, Phosphocarb, Phoxim, Pirimiphos (- methyl/- ethyl) , Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion

2. Natrium-Kanal-Modulatoren / Spannungsabhängige Natrium-Kanal-Blocker

2.1 Pyrethroide, zum Beispiel
Acrinathrin, Allethrin (d- cis- trans, d- trans) , Beta- Cyfluthrin, Bifenthrin, Bioallethrin, Bioallethrin- S- cyclopentylisomer, Bioethanomethrin, Biopermethrin, Bioresmethrin, Chlovaporthrin, Cis- Cypermethrin, Cis- Resmethrin, Cis- Permethrin, Clocythrin, Cycloprothrin, Cyfluthrin, Cyhalothrin, Cypermethrin (alpha-, beta-, theta-, zeta- ) , Cyphenothrin, Deltamethrin, Empenthrin (IR- isomer) , Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma- Cyhalothrin, Imiprothrin, Kadethrin, Lambda- Cyhalothrin, Metofluthrin, Permethrin (cis-, trans- ) , Phenothrin (1R- trans isomer) , Prallethrin, Profluthrin, Protrifenbute, Pyresmethrin, Resmethrin, RU 15525, Silafluofen, Tau- Fluvalinate, Tefluthrin, Terallethrin, Tetramethrin (- 1R- isomer) , Tralomethrin, Transfluthrin, ZXI 8901, Pyrethrins (pyrethrum)
DDT

2.2 Oxadiazine, zum Beispiel Indoxacarb

3. Acetylcholin- Rezeptor- Agonisten/- Antagonisten

3.1 Chloronicotinyle, zum Beispiel
Acetamiprid, Clothianidin, Dinotefuran, Imidacloprid, Nitenpyram, Nithiazine, Thiacloprid, Thiamethoxam

3.2 Nicotine, Bensultap, Cartap

4. Acetylcholin-Rezeptor-Modulatoren

4.1 Spinosyne, zum Beispiel Spinosad

5, GABA-gesteuerte Chlorid-Kanal-Antagonisten

5.1 Cyclodiene Organochlorine, zum Beispiel
Camphechlor, Chlordane, Endosulfan, Gamma-HCH, HCH, Heptachlor, Lindane, Methoxychlor

5.2 Fiprole, zum Beispiel
Acetoprole, Ethiprole, Fipronil, Vaniliprole

6. Chlorid-Kanal-Aktivatoren

6.1 Mectine, zum Beispiel
Avermectin, Emamectin, Emamectin-benzoate, Ivermectin, Milbemycin

7. Juvenilhormon-Mimetika, zum Beispiel
Diofenolan, Epofenonane, Fenoxycarb, Hydroprene, Kinoprene, Methoprene, Pyriproxifen, Triprene
8. Ecdysonagonisten/disruptoren

8.1 Diacylhydrazine, zum Beispiel
Chromafenozide, Halofenozide, Methoxyfenozide, Tebufenozide

9. Inhibitoren der Chitinbiosynthese

9.1 Benzoylharnstoffe, zum Beispiel
Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
9.2 Buprofezin
9.3 Cyromazine

10. Inhibitoren der oxidativen Phosphorylierung, ATP-Disruptoren

10.1 Diafenthiuron

10.2 Organotine, zum Beispiel Azocyclotin, Cyhexatin, Fenbutatin-oxide

11. Entkoppler der oxidativen Phoshorylierung durch Unterbrechung des H-Protongradienten

11.1 Pyrrole, zum Beispiel Chlorfenapyr
11.2 Dinitrophenole, zum Beispiel Binapacyrl, Dinobuton, Dinocap, DNOC

12. Seite-I -Elektronentransportinhibitoren

12.1 METI's, zum Beispiel Fenazaquin, Fenpyroximate, Pyrimidifen, Pyridaben, Tebufenpyrad, Tolfenpyrad
12.2 Hydramethylnon
12.3 Dicofol

13. Seite-II- Elektronentransportinhibitoren
Rotenone
14. Seite- III- Elektronentransportinhibitoren
Acequinocyl, Fluacrypyrim
15. Mikrobielle Disruptoren der Insektendarmmembran
Bacillus thuringiensis-Stämme
16. Inhibitoren der Fettsynthese
Tetronsäuren, zum Beispiel
Spirodiclofen, Spiromesifen
Tetramsäuren, zum Beispiel
3- (2, 5- Dimethylphenyl)- 8- methoxy- 2- oxo- 1- azaspiro [4.5] dec- 3- en- 4- yl ethyl carbonate (alias: Carbonic acid, 3- (2, 5- dimethylphenyl)- 8- methoxy- 2- oxo- 1- azaspiro [4.5] dec- 3- en- 4- yl ethyl ester, CAS- Reg.- No.: 382608- 10- 8) and Carbonic acid, cis- 3- (2, 5- dimethylphenyl)- 8- methoxy- 2- oxo- 1- azaspiro [4.5] dec- 3- en- 4- yl ethyl ester (CAS- Reg.- No.: 203313- 25- 1)
17, Carboxamide, zum Beispiel Flonicamid
18. Oktopaminerge Agonisten, zum Beispiel Amitraz
19. Inhibitoren der Magnesium-stimulierten ATPase, zum Beispiel Propargite
20. BDCAs, zum Beispiel N2- [1, 1- Dimethyl- 2- (methylsulfonyl) ethyl]- 3- iodo- N1- [2- methyl- 4- [1, 2, 2, 2-tetrafluoro- 1- (trifluoromethyl) ethyl] phenyl]- 1, 2- benzenedicarboxamide (CAS- Reg.- No.: 272451- 65- 7)
21. Nereistoxin-Analoge, zum Beispiel Thiocyclam hydrogen oxalate, Thiosultap-sodium
22. Biologika, Hormone oder Pheromone, zum Beispiel
Azadirachtin, Bacillus spec., Beauveria spec., Codlemone, Metarrhizium spec., Paecilomyces spec., Thuringiensin, Verticillium spec.
23. Wirkstoffe mit unbekannten oder nicht spezifischen Wirkmechanismen

23.1 Begasungsmittel, zum Beispiel
Aluminium phosphide, Methyl bromide, Sulfuryl fluoride
23.2 Selektive Fraßhemmer, zum Beispiel
Cryolite, Flonicamid, Pymetrozine
23.3 Milbenwachstumsinhibitoren, zum Beispiel
Clofentezine, Etoxazole, Hexythiazox
23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene, Verbutin, Verticillium lecanii,

WL- 108477, WL- 40027,
YI- 5201, YI- 5301, YI- 5302,
XMC, Xylylcarb,
ZA- 3274, Zeta- Cypermethrin, Zolaprofos, ZXI- 8901,
die Verbindung 3- Methyl- phenyl- propylcarbamat (Tsumacide Z) ,
die Verbindung 3- (5- Chlor- 3- pyridinyl)- 8- (2, 2, 2- trifluorethyl)- 8- azabicyclo [3.2.1] octan- 3- carbo- nitril (CAS- Reg.- Nr. 185982- 80- 3) und das entsprechende 3- endo- Isomere (CAS- Reg.- Nr. 185984- 60- 5) (vgl. WO- 96/37494, WO- 98/25923) ,
sowie Präparate, welche insektizid wirksame Pflanzenextrakte, Nematoden, Pilze oder Viren enthalten.

Auch eine Mischung mit anderen bekannten Wirkstoffen, wie Herbiziden, Düngemitteln, Wachstumsregulatoren, Safenern oder Semiochemicals ist möglich.

Die erfindungsgemäßen Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Die erfindungsgemäßen Wirkstoffkombinationen können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits") , die sowohl durch konventionelle Züchtung, durch Mutagenese oder durch rekombinante DNA- Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigen- schaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/ oder höherer Emährungswert der Ernteprodukte, höhere Lagerfähigkeit und/ oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/ oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis) , Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA (a) , CryIA (b) , CryIA (c) , CryIIA, CryIIIA, CryIIIB2, Cry9c Cry2Ab, Cry3Bb und CryIF sowie deren Kombinationen) in den Pflanzen erzeugt werden (im folgenden "Bt Pflanzen") . Als Eigenschaften ("Traits") werden auch besonders hervorgehoben die erhöhte Abwehr von Pflanzen gegen Pilze, Bakterien und Viren durch Systemische Akquirierte Resistenz (SAR) , Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"- Gen) . Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja) , KnockOut® (z.B. Mais) , StarLink® (z.B. Mais) , Bollgard® (Baumwolle) , Nucotn® (Baumwolle) und NewLeaf® (Kartoffel) vertrieben werden. Als Beispiele für Herbizid- tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja) , Liberty Link® (Toleranz gegen Phosphinotricin, z.B. Raps) , IMI® (Toleranz gegen Imidazolinone) und STS® (Toleranz gegen Sulfonylharnstoffe z.B. Mais) vertrieben werden. Als Herbizid resistente (konventionell auf Herbizid- Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits") .

Die aufgeführten Pflanzen können besonders vorteilhaft erfindungsgemäß mit den erfindungsgemäßen Wirkstoffmischungen behandelt werden. Die bei den Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffkombinationen wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffkombinationen der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffkombinationen geschieht im Veterinärsektor in bekannter Weise durch enterale Verabreichung in Form von beispielsweise Tabletten, Kapseln, Tränken, Drenchen, Granulaten, Pasten, Boli, des feed- through- Verfahrens, von Zäpfchen, durch parenterale Verabreichung, wie zum Beispiel durch Injektionen (intramuskulär, subcutan, intravenös, intraperitonal u.a.) , Implantate, durch nasale Applikation, durch dermale Anwendung in Form beispielsweise des Tauchens oder Badens (Dippen) , Sprühens (Spray) , Aufgießens (Pour-on und Spot- on) , des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffkombinationen als Formulierungen (beispielsweise Pulver, Emulsionen, fließfähige Mittel), die die Wirkstoffe in einer Menge von 1 bis 80 Gew.% enthalten, direkt oder nach 100 bis 10 000-facher Verdünnung anwenden oder sie als chemisches Bad verwenden.

Außerdem wurde gefunden, dass die erfindungsgemäßen Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillo-sum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittel, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des

Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise $\alpha$-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungs-mitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher (gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di- (2- ethylhexyl)- adipat, Stearate wie Butylstearat oder Amylstearat, Oleate wie Butyloleat, Glycerinether oder höhermolekulare Glykolether, Glycerinester sowie p- Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI- 25, Flufenoxuron, Hexaflumuron, Transfluthrin, Thiacloprid, Methoxyfenozide , Triflumuron, Clothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3- Iod- 2- propinyl- butylcarbamat, N- Octyl- isothiazolin- 3- on und 4, 5- Dichlor- N- octylisothiazolin- 3- on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln) , wie verschiedene Lepas- und Scalpellum- Arten, oder durch Arten der Gruppe Balanomorpha (Seepocken) , wie Balanus- oder Pollicipes- Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)- Wirkung aufweisen.

Durch Einsatz von erfindungsgemäßen Verbindungen allein oder in Kombination mit anderen Wirkstoffen, kann auf den Einsatz von Schwermetallen wie z.B. in Bis (trialkylzinn)- sulfiden, Tri- *n*- butylzinnlaurat, Tri- *n*- butylzinnchlorid, Kupfer (I)- oxid, Triethylzinnchlorid, Tri- *n*- butyl (2- phenyl- 4- chlorphenoxy)- zinn, Tributylzinnoxid, Molybdändisulfid, Antimonoxid, polymerem Butyltitanat, Phenyl- (bispyridin)- wismutchlorid, Tri- *n*- butylzinnfluorid, Manganethylenbisthiocarbamat, Zinkdimethyldithiocarbamat, Zinkethylenbisthiocarbamat, Zink- und Kupfersalze von 2- Pyridinthiol- 1- oxid, Bisdimethyldithiocarbamoylzinkethylenbisthiocarbamat, Zinkoxid, Kupfer (I)- ethylen- bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling- Mittel eignen sich vorzugsweise:
Algizide wie 2- *tert*.- Butylamino- 4- cyclopropylamino- 6- methylthio- 1, 3, 5- triazin, Dichlorophen, Diuron, Endothal, Fentinacetat, Isoproturon, Methabenzthiazuron, Oxyfluorfen, Quinoclamine und Terbutryn;
Fungizide wie Benzo [*b*] thiophencarbonsäurecyclohexylamid- S, S- dioxid, Dichlofluanid, Fluor- folpet, 3- Iod- 2- propinylbutylcarbamat, Tolylfluanid und Azole wie Azaconazole, Cyproconazole, Epoxyconazole, Hexaconazole, Metconazole, Propiconazole und Tebuconazole;
Molluskizide wie Fentinacetat, Metaldehyd, Methiocarb, Niclosamid, Thiodicarb und Trimethacarb, Fe- chelate,
oder herkömmliche Antifouling- Wirkstoffe wie 4, 5- Dichlor- 2- octyl- 4- isothiazolin- 3- on, Diiod- methylparatrylsulfon, 2- (N, N- Dimethylthiocarbamoylthio)- 5- nitrothiazyl, Kalium-, Kupfer-, Natrium- und Zinksalze von 2- Pyridinthiol- 1- oxid, Pyridin- triphenylboran, Tetrabutyldistannoxan, 2, 3, 5, 6- Tetrachlor- 4- (methylsulfonyl)- pyridin, 2, 4, 5, 6- Tetrachloroisophthalonitril, Tetramethylthiuramdisulfid und 2, 4, 6- Trichlorphenylmaleinimid.

Die verwendeten Antifouling-Mittel enthalten den erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling- Anstrichmittel enthalten neben den algiziden, fungiziden, molluskiziden und er- findungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/ Vinylacetat- Copolymersysteme in Form wässriger Dispersionen oder in Form von organischen Lösungsmittelsystemen, Butadien/Sty- rol/ Acrylnitril- Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self- Polishing- Antifouling- Systemen können die erfindungsgemäßen Verbindungen oder die oben genannten Mischungen eingearbeitet werden.

Die Wirkstoffkombinationen eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellerge-triebenen Verdampfern, energielosen bzw. passiven Verdampfungssystemen, Mottenpapieren, Mottensäckchen und Mottengelen, als Granulate oder Stäube, in Streuködem oder Köderstationen.

Die gute Wirkung der erfindungsgemäßen Wirkstoffkombinationen geht aus den nachfolgenden Beispielen hervor. Während die einzelnen Wirkstoffe in der Wirkung Schwächen aufweisen, zeigen die Kombinationen eine Wirkung, die über eine einfache Wirkungssummierung hinausgeht.

Ein synergistischer Effekt liegt bei Insektiziden und Akariziden immer dann vor, wenn die Wirkung der Wirkstoffkombinationen größer ist als die Summe der Wirkungen der einzeln applizierten Wirkstoffe.

Die zu erwartende Wirkung für eine gegebene Kombination zweier Wirkstoffe kann nach S.R. Colby, Weeds 15 (1967), 20-22) wie folgt berechnet werden:

Wenn

X den Abtötungsgrad, ausgedrückt in % der unbehandelten Kontrolle, beim Einsatz des Wirkstoffes A in einer Aufwandmenge von m g/ha oder in einer Konzentration von m ppm bedeutet,

Y den Abtötungsgrad, ausgedrückt in % der unbehandelten Kontrolle, beim Einsatz des Wirkstoffes B in einer Aufwandmenge von n g/ha oder in einer Konzentration von n ppm bedeutet und

E den Abtötungsgrad, ausgedrückt in % der unbehandelten Kontrolle, beim Einsatz der Wirkstoffe A und B in Aufwandmengen von m und n g/ha oder in einer Konzentration von m und n ppm bedeutet,

dann ist

$$E = X + Y - \frac{X \cdot Y}{100}$$

Ist der tatsächliche insektizide Abtötungsgrad größer als berechnet, so ist die Kombination in ihrer Abtötung überadditiv, d.h. es liegt ein synergistischer Effekt vor. In diesem Fall muss der tatsächlich beobachtete Abtötungsgrad größer sein als der aus der oben angeführten Formel errechnete Wert für den erwarteten Abtötungsgrad (E).

**Beispiel A** (nicht erfindungsgemäß)

**Aphis gossypii -Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter (*Gossypium hirsutum*), die stark von der Baumwollblattlaus (*Aphis gossypii*) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden. Die ermittelten Abtötungswerte verrechnet man nach der Colby-Formel (siehe Blatt 1).

Bei diesem Test zeigt z. B. die folgende Wirkstoffkombination gemäß vorliegender Anmeldung eine synergistisch verstärkte Wirksamkeit im Vergleich zu den einzeln angewendeten Wirkstoffen:

**Tabelle A**

Pflanzenschädigende Insekten

**Aphis gossypii - Test**

| Wirkstoff | Konzentration in ppm | Abtötung in % nach 1$^d$ | |
|---|---|---|---|
| **Beta-cyfluthrin** | 0,12 | | 5 |
| **Thiacloprid** | 0,6 | | 35 |
| **Beta-cyfluthrin + Thiacloprid (1:5)** | | gef.* | ber.** |
| | 0,12 + 0,6 | 95 | 38,25 |

* gef. = gefundene Wirkung
** ber. = nach der Colby-Formel berechnete Wirkung

**Beispiel B**

**Heliothis armigera - Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter (*Gossypium hirsutum*) werden durch Tauchen in die Wirkstoffzubereitung der gewünschten Konzentration behandelt und mit Raupen des Baumwollkapselwurms (*Heliothis armigera*) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel (siehe Blatt 1).

Bei diesem Test zeigte die folgende Wirkstoffkombination gemäß vorliegender Anmeldung eine synergistisch verstärkte Wirksamkeit im Vergleich zu den einzeln angewendeten Wirkstoffen:

**Tabelle B**

Pflanzenschädigende Insekten

**Heliothis armigera Test**

| Wirkstoff | Konzentration in ppm | Abtötungin in % nach $6^d$ | |
|---|---|---|---|
| **Beta-cyfluthrin** | 0,12 | 0 | |
| **Thiacloprid** | 0,6 | 0 | |
| **Beta-cyfluthrin + Thiacloprid (1:5) nicht erfindungsgemäß** | | gef.* | ber.** |
| | 0,12 + 0,6 | 100 | 0 |
| **Lambda-cyhalothrin** | 0,12 | 55 | |
| **Thiacloprid** | 0,12 | 0 | |
| **Lambda-cyhalothrin + Thiacloprid (1:1)** erfindungsgemäß | | gef.* | ber.** |
| | 0,12 + 0,12 | 100 | 55 |

* gef. = gefundene Wirkung

** ber. = nach der Colby-Formel berechnete Wirkung

**Beispiel C** (nicht erfindungsgemäß)

**Myzus persicae -Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*), die stark von der Grünen Pfirsichblattlaus (*Myzus persicae*) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden. Die ermittelten Abtötungswerte verrechnet man nach der Colby-Formel (siehe Blatt 1).

Bei diesem Test zeigt z. B. die folgende Wirkstoffkombination gemäß vorliegender Anmeldung eine synergistisch verstärkte Wirksamkeit im Vergleich zu den einzeln angewendeten Wirkstoffen:

**Tabelle C**
Pflanzenschädigende Insekten
**Myzus persicae - test**

| Wirkstoff | Konzentration in ppm | Abtötung in % nach 6^d | |
|---|---|---|---|
| **Beta-Cyfluthrin** | 0,12 | 0 | |
| **Thiacloprid** | 0,6 | 35 | |
| **Beta-Cyfluthrin + Thiacloprid (1:5)** | | gef.* | ber.** |
| | 0,12 + 0,6 | 95 | 35 |

* gef. = gefundene Wirkung
** ber. = nach der Colby-Formel berechnete Wirkung

**Tabelle C**
Pflanzenschädigende Insekten
**Myzus persicae - test**

| Wirkstoff | Konzentration in ppm | Abtötung in % nach 6^d | |
|---|---|---|---|
| **Bifenthrin** | 0,16 | 0 | |
| **Thiacloprid** | 0,8 | 0 | |
| **Bifenthrin + Thiacloprid (1:5)** | | gef.* | ber.** |
| | 0,16 + 0,8 | 40 | 0 |

* gef. = gefundene Wirkung
** ber. = nach der Colby-Formel berechnete Wirkung

**Tabelle C**
Pflanzenschädigende Insekten
**Myzus persicae - test**

| Wirkstoff | Konzentration in ppm | Abtötung in % nach 6^d | |
|---|---|---|---|
| **Gamma-Cyhalothrin** | 0,032 | 0 | |
| **Thiacloprid** | 0,8 | 10 | |
| **Gamma-Cyhalothrin + Thiacloprid (1:25)** | | gef.* | ber.** |
| | 0,032 + 0,8 | 35 | 10 |

* gef. = gefundene Wirkung
** ber. = nach der Colby-Formel berechnete Wirkung

**Beispiel D**

**Phaedon cochleariae - Larven -Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Larven des Meerrettichblattkäfers (*Phaedon cochleariae*) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden. Die ermittelten Abtötungswerte verrechnet man nach der Colby-Formel (siehe Blatt 1).

Bei diesem Test zeigte die folgende Wirkstoffkombination gemäß vorliegender Anmeldung eine synergistisch verstärkte Wirksamkeit im Vergleich zu den einzeln angewendeten Wirkstoffen:

Tabelle D
Pflanzenschädigende Insekten
**Phaedon cochleariae Larven - Test**

| Wirkstoff | Konzentration in ppm | Abtötung in % nach 6[d] | |
|---|---|---|---|
| **Beta-cyfluthrin** | 0,12 | 15 | |
| **Thiacloprid** | 3 | 15 | |
| **Beta-cyfluthrin** + **Thiacloprid (1:25)** nicht erfindungsgemäß | | gef.* | ber.** |
| | 0,12 + 3 | 100 | 27,75 |

* gef. = gefundene Wirkung
** ber. = nach der Colby-Formel berechnete Wirkung

Tabelle D
Pflanzenschädigende Insekten
**Phaedon cochleariae Larven - Test**

| Wirkstoff | Konzentration in ppm | Abtötung in % nach 6[d] | |
|---|---|---|---|
| **Alpha-Cypermethrin** | 0,16 | 5 | |
| **Thiacloprid** | 4 | 5 | |
| **Alpha-Cypermethrin** + **Thiacloprid (1:25)** erfindungsgemäß | | gef.* | ber.** |
| | 0,16 + 4 | 55 | 9,75 |

* gef. = gefundene Wirkung
** ber. = nach der Colby-Formel berechnete Wirkung

Tabelle D
Pflanzenschädigende Insekten
**Phaedon cochleariae Larven - Test**

| Wirkstoff | Konzentration in ppm | Abtötung in % nach 6[d] | |
|---|---|---|---|
| **Bifenthrin** | 0,8 | 35 | |
| **Thiacloprid** | 4 | 30 | |
| **Bifenthrin + Thiacloprid (1:5)** nicht erfindungsgemäß | | gef.* | ber.** |
| | 0,8 + 4 | 100 | 54,5 |

* gef. = gefundene Wirkung
** ber. = nach der Colby-Formel berechnete Wirkung

Tabelle D
Pflanzenschädigende Insekten
**Phaedon cochleariae Larven - Test**

| Wirkstoff | Konzentration in ppm | Abtötung in % nach 6[d] | |
|---|---|---|---|
| **Deltamethrin** | 0,16 | 30 | |
| **Thiacloprid** | 20 | 40 | |
| **Deltamethrin** + **Thiacloprid (1:125)** erfindungsgemäß | | gef.* | ber.** |

(fortgesetzt)

Pflanzenschädigende Insekten

**Phaedon cochleariae Larven - Test**

| Wirkstoff | Konzentration in ppm | Abtötung in % nach 6[d] | |
|---|---|---|---|
| | 0,16 + 20 | 90 | 58 |
| * gef. = gefundene Wirkung<br>** ber. = nach der Colby-Formel berechnete Wirkung | | | |

## Beispiel E

**Plutella xylostella - Test (sensibler Stamm)**

Lösungsmittel: 7 Gewichtsteile Dimethylformamid
Emulgator: 2 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen der Kohlschabe (*Plutella xylostella,* sensibler Stamm) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel (siehe Blatt 1).

Bei diesem Test zeigte die folgende Wirkstoffkombination gemäß vorliegender Anmeldung eine synergistisch verstärkte Wirksamkeit im Vergleich zu den einzeln angewendeten Wirkstoffen:

**Tabelle E**

Pflanzenschädigende Insekten

**Plutella xylostella - Test (normal sensibel)**

| Wirkstoff | Konzentration in ppm | Abtötung in % nach 6[d] | |
|---|---|---|---|
| **Beta-cyfluthrin** | 0,024 | 10 | |
| **Thiacloprid** | 0,6 | 0 | |
| **Beta-cyfluthrin + Thiacloprid (1:25)** nicht erfindungsgemäß | | gef.* | ber.** |
| | 0,024 + 0,6 | 40 | 10 |
| **Lambda-cyhalothrin** | 0,024 | 40 | |
| **Thiacloprid** | 0,6 | 0 | |
| **Lambda-cyhalothrin + Thiacloprid (1:25)** erfindungsgemäß | | gef.* | ber.** |
| | 0,024 + 6 | 80 | 40 |
| * gef. = gefundene Wirkung<br>** ber. = nach der Colby-Formel berechnete Wirkung | | | |

## Beispiel F

**Plutella xylostella - Test (resistenter Stamm)**

Lösungsmittel: 7 Gewichtsteile Dimethylformamid
Emulgator: 2 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Raupen der Kohlschabe (*Plutella xylostella,* resistenter Stamm) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel (siehe Blatt 1).

Bei diesem Test zeigte die folgende Wirkstoffkombination gemäß vorliegender Anmeldung eine synergistisch verstärkte Wirksamkeit im Vergleich zu den einzeln angewendeten Wirkstoffen:

**Tabelle F**

Pflanzenschädigende Insekten

**Plutella xylostella - Test (resistent)**

| Wirkstoff | Konzentration in ppm | Abtötung in % nach 6[d] | |
|---|---|---|---|
| **Beta-cyfluthrin** | 0,6 | 35 | |
| **Thiacloprid** | 3 | 0 | |
| **Beta-cyfluthrin + Thiacloprid (1:5)** nicht erfindungsgemäß | | <u>gef</u>.* | <u>ber</u>.** |
| | 0,6 + 3 | 85 | 35 |
| **Lambda-cyhalothrin** | 0,6 | 40 | |
| **Thiacloprid** | 15 | 10 | |
| **Lambda-cyhalothrin + Thiacloprid (1:25)** erfindungsgemäß | | <u>gef</u>.* | <u>ber</u>.** |
| | 0,6 + 15 | 85 | 46 |

* gef. = gefundene Wirkung
** ber. = nach der Colby-Formel berechnete Wirkung

<u>**Beispiel G**</u> (nicht erfindungsgemäß)

**Spodoptera exigua - Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Raupen der Zuckerrübeneule (*Spodoptera exigua*) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel (siehe Blatt 1).

Bei diesem Test zeigte die folgende Wirkstoffkombination gemäß vorliegender Anmeldung eine synergistisch verstärkte Wirksamkeit im Vergleich zu den einzeln angewendeten Wirkstoffen:

**Tabelle G**

Pflanzenschädigende Insekten

**Spodoptera exigua - Test**

| Wirkstoff | Konzentration in ppm | Abtötung in % nach 6[d] |
|---|---|---|
| **Beta-cyfluthrin** | 0,6 | 25 |

(fortgesetzt)

Pflanzenschädigende Insekten

**Spodoptera exigua - Test**

| Wirkstoff | Konzentration in ppm | Abtötung in % nach $6^d$ | |
|---|---|---|---|
| **Thiacloprid** | 15 | 0 | |
| **Beta-cyfluthrin + Thiacloprid (1:25)** | | gef.* | ber.** |
| | 0,6 + 15 | 100 | 25 |

* gef. = gefundene Wirkung
** ber. = nach der Colby-Formel berechnete Wirkung

**Beispiel H** (nicht erfindungsgemäß)

**Spodoptera frugiperda - Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (*Brassica oleracea*) werden durch Tauchen in die Wirkstoffzubereitung der gewünschten Konzentration behandelt und mit Raupen des Heerwurms (*Spodoptera frugiperda*) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel (siehe Blatt 1).

Bei diesem Test zeigte die folgende Wirkstoffkombination gemäß vorliegender Anmeldung eine synergistisch verstärkte Wirksamkeit im Vergleich zu den einzeln angewendeten Wirkstoffen:

**Tabelle H**

Pflanzenschädigende Insekten

**Spodoptera frugiperda - Test**

| Wirkstoff | Konzentration in ppm | Abtötung in % nach $3^d$ | |
|---|---|---|---|
| **Beta-cyfluthrin** | 0,12 | 5 | |
| **Thiacloprid** | 0,6 | 0 | |
| **Beta-cyfluthrin** + **Thiacloprid (1:5)** | | gef.* | ber.** |
| | 0,12 + 0,6 | 100 | 5 |

* gef. = gefundene Wirkung
** ber. = nach der Colby-Formel berechnete Wirkung

## Patentansprüche

**1.** Wirkstoffkombinationen enthaltend Thiacloprid und mindestens eine der nachfolgenden Verbindungen
Alpha-Cypermethrin
Deltamethrin
Lambda-Cyhalothrin
Tau-fluvalinat
Zeta-Cypermethrin

## Claims

1. Active compound combinations comprising thiacloprid and at least one of the compounds below
alpha-cypermethrin
deltamethrin
lambda-cyhalothrin
tau-fluvalinate
zeta-cypermethrin.

## Revendications

1. Associations de substances actives contenant du thiaclopride et au moins l'un des composés suivants
alpha-cyperméthrine
deltaméthrine
lambda-cyhalothrine
tau-fluvalinate
zêta-cyperméthrine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0235725 A
- WO 9322297 A
- WO 9310083 A
- DE 2641343 A
- EP 347488 A
- EP 210487 A
- US 3264177 A
- EP 234045 A
- WO 0243494 A
- WO 02087338 A
- WO 0230200 A
- FR 2784011 A
- US 5661164 A
- EP 067461 A
- DE 2326077 A
- EP 106469 A
- EP 038617 A
- EP 026542 A
- WO 9637494 A
- WO 9825923 A
- WO 9429268 A


### In der Beschreibung aufgeführte Nicht-Patentliteratur


- **UNGERER.** *Chem. Ind.,* 1985, vol. 37, 730-732
- **WILLIAMS.** Antifouling Marine Coatings. Noyes, Park Ridge, 1973
- **S.R. COLBY.** *Weeds,* 1967, vol. 15, 20-22